# EUROPEAN PATENT APPLICATION

(11) **EP 3 936 624 A1**
(43) Date of publication of application: **12.01.2022**
(21) Application number: 20768993.6
(22) Date of filing: 06.03.2020
(51) Int. Cl.: C12Q 1/6881, G01N 33/569

(54) **MARKER FOR PREDICTING TUMOR REACTIVITY OF LYMPHOCYTES, AND USE THEREOF**

(30) Priority: 08.03.2019 KR 20190026918
(71) Applicant: Neogentc Corp., Seoul 05505 (KR)
(72) Inventor: LEE, Hee Jin, Seoul 06003 (KR); GONG, Gyungyub, Gyeonggi-do 13449 (KR); LEE, Hee Jae, Chungcheongnam-do 31194 (KR); SEO, Jeong Han, Seoul 05382 (KR); LIM, Gyeongback, Seoul 05376 (KR)
(74) Representative: Schön, Christoph
(86) International application number: PCT/KR2020/003157
(87) International publication number: WO 2020/184911

(57) **Abstract**

The present invention relates to a marker for predicting a tumor reactivity of lymphocytes, a composition for predicting a tumor reactivity, and a method for predicting a tumor reactivity. Using the genetic marker according to the present invention, a predictive model that can predict the tumor reactivity of cultured lymphocytes may be constructed, and it is possible to more accurately select tumor-specific lymphocytes to produce an effective immunotherapeutic agent, by predicting the reactivity of the lymphocytes through the present invention. In addition, by using the above genetic markers, it is possible to more conveniently and non-invasively separate lymphocytes from body tissues, blood, or body fluids, deviating from the conventional invasive methods, and to select only lymphocytes with tumor-specific activity, for immunotherapy. It is expected to widely use with various application.

## Description

### [Technical Field]

The present invention relates to a marker for predicting tumor reactivity of lymphocytes, a composition for predicting tumor reactivity, and a method of predicting tumor reactivity.

### [Background Art]

Recently, as the importance of immune factors in tumors has been revealed, treatment methods using immune-related factors are being actively developed. For example, immunotherapy includes vaccines targeting tumor-related antigens, monoclonal antibodies preventing T cell exhaustion caused by overexpression of an inhibitory receptor, or use of a tumor necrosis factor (TNF) receptor superfamily activating T lymphocytes by activating co-stimulatory receptors for T lymphocytes, treatment targeting an immunoinhibitory factor, and cell therapy using natural killer cells (NK cells) or tumor-specific T lymphocytes. Recently, it has been revealed that immunotherapy using an antibody recognizing an immunoinhibitory factor (immune checkpoint inhibitor, anti-PD1 or anti-CTLA4 antibody) is effective in tumors such as malignant melanoma. However, since an antibody-based therapy targets an antigen located on a cell surface, it has the disadvantage in that it cannot be applied to antigens in cells.

As the importance of immune factors in tumors in cell therapy using NK cells or tumor-specific T lymphocytes has been recently revealed, treatment methods using immune-related factors are being actively developed. For example, immunotherapy includes vaccines targeting tumor-related antigens, monoclonal antibodies preventing T cell exhaustion caused by overexpression of an inhibitory receptor, or the use of a tumor necrosis factor (TNF) receptor superfamily activating T lymphocytes by activating co-stimulatory receptors for T lymphocytes, treatment targeting an immunoinhibitory factor, and cell therapy using natural killer cells (NK cells) or tumor-specific T lymphocytes. Recently, it has been revealed that immunotherapy using an antibody recognizing an immunoinhibitory factor (immune checkpoint inhibitor, anti-PD1 or anti-CTLA4 antibody) is effective in tumors such as malignant melanoma. However, since an antibody-based therapy targets an antigen located on a cell surface, it has the disadvantage in that it cannot be applied to antigens in cells.

Cell therapy using NK cells or tumor-specific T lymphocytes is called immunomodulatory cell therapeutics, and this is a therapeutic method used for the purpose of treating diseases by activating the immune response in the body using immune cells. Immunomodulatory cell therapeutics is being developed mainly for cancer treatment, and since it has a therapeutic effect by activating immune functions by direct administration of immune cells into a patient, it is a field that is expected to account for the major part of future biopharmaceuticals due to its treatment mechanism and efficacy that is distinguished from conventional surgical therapy, chemotherapy, and radiation therapy, which have been sued for cancer treatment.

Broadly, there are several types of immunomodulatory cell therapeutics, such as lymphokine-activated killer (LAK) cells, dendritic cells, and T cell-based therapeutics, and the T cell-based cell therapy includes, representatively, tumor-infiltrating lymphocytes (TILs) produced by proliferation of T cells which is infiltrated into a patient's tumor tissue; T cell receptor-expressing T cells (TCR-modified T cells; TCR-T) introducing a gene of a T cell receptor (TCR) or a chimeric antigen receptor (CAR) after isolating T cells of a patient; and chimeric antigen receptor-expressing T cells (CAR-modified T cells; CAR-Ts).

Tumor-infiltrating lymphocytes (TILs) are lymphocytes present in tumor tissue, which are obtained by isolating T cells with tumor cell killing capacity from a patient's tumor tissue, proliferating the T cells and then re-administering them into the patient, and this therapy is studied mainly for solid carcinoma capable of being biopsied. TIL is the first immunomodulatory cell therapeutics using T cells that was studied for clinical efficacy, and Dr. Steven Rosenberg and his team at the National Cancer Institute in the United States first reported in 1988 that TIL administration into patients with metastatic melanoma shows an anticancer effect, and there has been no report on serious side effects from TIL administration to date. However, TIL has a technical disadvantage in that the process of isolating tumor-specific T cells from tumor tissue is complicated, and has a low yield. In addition, similar to the case of LAK, it is co-administered with interleukin-2 (IL-2) for boosting anticancer efficacy, and thus has side effects caused by IL-2 administered at a high concentration, which is a problem to be solved (Science. 2015 Apr 3;348 (6230):62-8.).

Therefore, to overcome the limitation of the above-described conventional cell therapeutics using TIL and develop more effective T cell immune cell therapeutics, the inventors made an effort to find a genetic marker capable of efficiently selecting only lymphocytes exhibiting a tumor-specific activity. The genetic marker was found from lymphocytes isolated from the body, not limited to tumor tissue, by non-invasive method and cultured. As a result, the inventors discovered 17 markers and completed the present invention based on them.

### [Disclosure]

### [Technical Problem]

To find a genetic marker capable of effectively selecting lymphocytes exhibiting tumor-specific reactivity, The present inventors discovered a total of 17 genetic biomarkers using lymphocytes isolated from tumor tissues of breast cancer patients and verified their effectiveness. As a result of this effort, the present inventors have accomplished the present invention.

Therefore, the present invention is directed to providing a composition for predicting the tumor reactivity of lymphocytes.

However, technical problems to be solved in the present invention are not limited to the above-described problems, and other problems which are not described herein will be fully understood by those of ordinary skill in the art from the following descriptions.

### [Technical Solution]

To attain the purpose of the present invention, the present invention provides a composition for predicting the tumor reactivity of lymphocytes, which includes an agent capable of measuring a mRNA level or an agent capable of measuring a protein level for ITGA6 gene (GenBank Accession Nos.: NM_000210.4, NM_001079818.3, NM_001316306.2, NM_001365529.2 and NM_001365530.2).

In one embodiment of the present invention, the composition may also include an agent capable of measuring mRNA levels or an agent capable of measuring protein levels for one or more types of genes selected from the group consisting of ATP6V0A1 (GenBank Accession No.: NM_001130020.3, NM_001130021.3, NM_001378522.1, NM_001378523.1 and NM_001378530.1), ARRDC3 (GenBank Accession No.: NM_001329670.2, NM_001329671.2, NM_001329672.2 and NM_020801.4), CD23 (GenBank Accession No.: NM_001207019.2, NM_001220500.2 and NM_002002.4), CD200 (GenBank Accession No.: NM_001004196.3, NM_001318826.1, NM_001318828.1, NM_001318830.1 and NM_001365851.2), CD300C (GenBank Accession No.: NM_006678.5), CYSLTR1 (GenBank Accession No.: NM_001282186.1, NM_001282187.2, NM_001282188.2 and NM_006639.4), ITGB1 (GenBank Accession No.: NM_002211.4, NM_033668.2 and NM_133376.2), MBOAT2 (GenBank Accession No.: NM_001321265.2, NM_001321266.2, NM_001321267.2 and NM_138799.4), Met (GenBank Accession No.: NM_000245.4, NM_001127500.3, NM_001324401.2 and NM_001324402.2), MYO9A (GenBank Accession No.: NM_006901.4), PTPN13 (GenBank Accession No.: NM_006264.3, NM_080683.3, NM_080684.3 and NM_080685.2), S100P (GenBank Accession No.: NM_005980.3), SECTM1 (GenBank Accession No.: NM_003004.3), TCN2 (GenBank Accession No.: NM_000355.4 and NM_001184726.1), TSPAN2 (GenBank Accession No.: NM_001308315.1, NM_001308316.1 and NM_005725.6) and VSIG1 (GenBank Accession No.: NM_001170553.1 and NM_182607.5).

In addition, the present invention provides a composition for predicting the tumor reactivity of lymphocytes, which includes an agent capable of measuring mRNA levels or an agent capable of measuring protein levels for three or more types of genes selected from the group consisting of ATP6V0A1 (GenBank Accession No.: NM_001130020.3, NM 001130021.3, NM_001378522.1, NM_001378523.1 and NM_001378530.1), MBOAT2 (GenBank Accession No.: NM_001321265.2, NM_001321266.2, NM_001321267.2 and NM_138799.4), PTPN13 (GenBank Accession No.: NM_006264.3, NM_080683.3, NM_080684.3 and NM_080685.2), TCN2 (GenBank Accession No.: NM_000355.4 and NM_001184726.1) and TSPAN2 (GenBank Accession No.: NM_001308315.1, NM_001308316.1 and NM_005725.6).

In one embodiment of the present invention, the composition may also include an agent capable of measuring mRNA levels or an agent capable of measuring protein levels for ATP6V0A1, MBOAT2 and TSPAN2 genes.

In another embodiment of the present invention, the composition may include an agent capable of measuring mRNA levels or an agent capable of measuring protein levels for PTPN13, TCN2 and TSPAN2 genes.

In another embodiment of the present invention, the composition may also includes an agent capable of measuring mRNA levels or an agent capable of measuring protein levels for one or more genes selected from the group consisting of ARRDC3 (GenBank Accession No.: NM_001329670.2, NM_001329671.2, NM_001329672.2 and NM_020801.4), CD23 (GenBank Accession No.: NM_001207019.2, NM_001220500.2 and NM_002002.4), CD200 (GenBank Accession No.: NM_001004196.3, NM 001318826.1, NM_001318828.1, NM_001318830.1 and NM_001365851.2), CD300C (GenBank Accession No.: NM_006678.5), CYSLTR1 (GenBank Accession No.: NM_001282186.1, NM_001282187.2, NM_001282188.2 and NM_006639.4), ITGA6 (GenBank Accession No.: NM_000210.4, NM_001079818.3, NM_001316306.2, NM_001365529.2 and NM_001365530.2), ITGB1 (GenBank Accession No.: NM_002211.4, NM_033668.2 and NM_133376.2), Met (GenBank Accession No.: NM_000245.4, NM_001127500.3, NM_001324401.2 and NM_001324402.2), MYO9A (GenBank Accession No.: NM_006901.4), S100P (GenBank Accession No.: NM_005980.3), SECTM1 (GenBank Accession No.: NM_003004.3) and VSIG1 (GenBank Accession No.: NM_001170553.1 and NM_182607.5).

In another embodiment of the present invention, the lymphocytes may be isolated from tumor tissue, blood, or a body fluid.

In another embodiment of the present invention, the agent for measuring an mRNA level may be sense and anti-sense primers, or probes complementarily binding to mRNA of the gene.

In another embodiment of the present invention, the agent for measuring a protein level may be an antibody specifically binding to a protein encoded by the gene.

### [Advantageous Effects]

If only lymphocytes with tumor-specific activity can be selectively selected from various body-derived lymphocytes, based on this, an effective immunotherapeutic agent can be produced. Accordingly, a predictive model that can predict tumor reactivity of lymphocytes cultured using a genetic marker according to the present invention can be constructed, and an effective immunotherapeutic agent can be produced more precisely selecting tumor-specific lymphocytes by predicting the reactivity of lymphocytes using this model. In addition, by using the genetic marker, it can be possible to be used in immunotherapy by more simply isolating lymphocytes from tissue, blood or a body fluid in the body by a non-invasive method, rather than a conventional invasive method, and selecting only lymphocytes having tumor-specific activity, and thus it is expected to use the genetic marker in various applications.

### [Description of Drawings]

FIG. 1 shows the gene analysis results showing a significant correlation (p<0.05) by examining the correlation between the ratio of IFN-γ and the gene expression value by bivariate correlation analysis (Spearman Correlation), which is performed using the ratio of IFN-γ secreted when autologous breast cancer cells and TIL were co-cultured, compared to the control group consisting of only tumor infiltrating lymphocytes (TIL); and each gene expression in TIL responsive to tumor cells.
FIG. 2a shows the results of quantifying the difference in expression level according to reactivity of TILs with respect to each of six genes confirmed to have a significant correlation with the presence or absence of tumor-specific reactivity of TILs derived from a triple-negative breast cancer (TNBC) patient among 17 genes derived in Example 2.
FIG. 2b shows the results of quantifying the difference in expression level of ITGA6 according to tumor-specific reactivity in total TILs (CD45+), NKT cells and T cells, which are confirmed to have a significant correlation with ITGA6 expression among TIL types.
FIG. 3a is a result of main component analysis (PCA) for TILs derived from 15 TNBC patients.
FIG. 3b is a heatmap for TNBC patient-derived TILs, which shows expression levels of 17 genes selected in Example 2.
FIG. 3c is a biplot graph obtained with the PCA result of FIG. 3a, representing patient-derived TIL samples and 17 genes.
FIG. 3d is a heatmap showing expression levels of six genes which are shown to have a relationship with a group with reactivity according to the analysis result of FIG. 3c.
FIG. 4a shows a heatmap showing expression levels of gene combinations after each combination consisting of 2 to 17 genes and a variable thereof in consideration of the interaction between 17 genes are obtained, regression analysis is performed to identify a 10-gene combination showing the highest prediction accuracy, and variables of the 13 combinations are obtained.
FIG. 4b is a heatmap showing an expression level of each of genes consisting of the combination (No. 2) having the highest negative coefficient of the combination variables.
FIG. 4c is a heatmap showing an expression level of each of the genes consisting of the combination (No. 7) having the highest positive coefficient of the combination variables.
FIG. 4d is a heatmap for 8 genes, which are not common among genes constituting the combinations of FIGS. 4B and 4C.
FIGS. 5a and 5b show the ROC curves and the AUC value according to each gene combination, in which each gene combination is composed of 2 to 8 genes in consideration of the interaction between the 8 genes in FIG. 4d.
FIGS. 6a and 6b show ROC curves for 10 gene combination variables by confirming that a model consisting of three genes exhibits the greatest performance as a result of regression analysis and random forest machine learning analysis, and AUC values obtained therefrom.
FIGS. 6c and 6d show six combinations in which the AUC values are 0.7 or more from the results of FIGS. 6A and 6B, and ROC curves thereof.
FIG. 6e shows results of analyzing the reactivity prediction accuracy for tumors by performing Confusion Matrix analysis on the six combination variables.

### [Modes of the Invention]

The inventors had made an effort to discover a genetic marker capable of effectively selecting lymphocytes exhibiting tumor-specific activity using lymphocytes isolated from tumor tissue of a breast cancer patient, and thus found a total of 17 genes and verified their efficacy. Therefore, the present invention was completed.

Hereinafter, the present invention will be described in detail.

The present invention provides a marker composition for predicting the tumor reactivity of lymphocytes, which includes a mRNA or a protein for ITGA6 gene (GenBank Accession No.: NM_000210.4, NM_001079818.3, NM_001316306.2, NM_001365529.2 and NM_001365530.2).

The marker composition may also include mRNA or protein for one or more types of gene selected from the group consisting of ATP6V0A1 (GenBank Accession No.: NM_001130020.3, NM 001130021.3, NM_001378522.1, NM_001378523.1 and NM_001378530.1), ARRDC3 (GenBank Accession No.: NM_001329670.2, NM_001329671.2, NM_001329672.2 and NM_020801.4), CD23 (GenBank Accession No.: NM_001207019.2, NM_001220500.2 and NM_002002.4), CD200 (GenBank Accession No.: NM_001004196.3, NM_001318826.1, NM_001318828.1, NM_001318830.1 and NM_001365851.2), CD300C (GenBank Accession No.: NM_006678.5), CYSLTR1 (GenBank Accession No.: NM_001282186.1, NM_001282187.2, NM_001282188.2 and NM_006639.4), ITGB1 (GenBank Accession No.: NM_002211.4, NM_033668.2 and NM_133376.2), MBOAT2 (GenBank Accession No.: NM_001321265.2, NM_001321266.2, NM_001321267.2 and NM_138799.4), Met (GenBank Accession No.: NM_000245.4, NM_001127500.3, NM_001324401.2 and NM_001324402.2), MYO9A (GenBank Accession No.: NM_006901.4), PTPN13 (GenBank Accession No.: NM_006264.3, NM_080683.3, NM_080684.3 and NM_080685.2), S100P (GenBank Accession No.: NM_005980.3), SECTM1 (GenBank Accession No.: NM_003004.3), TCN2 (GenBank Accession No.: NM_000355.4 and NM_001184726.1), TSPAN2 (GenBank Accession No.: NM_001308315.1, NM_001308316.1 and NM_005725.6) and VSIG1 (GenBank Accession No.: NM_001170553.1 and NM_182607.5).

The present invention also provides a composition for predicting the tumor reactivity of lymphocytes, which includes an agent for measuring a mRNA level or an agent for measuring a protein level for ITGA6 gene, and a kit for predicting the tumor reactivity of lymphocytes, which includes the composition.

The composition may also include an agent for measuring mRNA levels or an agent for measuring protein levels for one or more types of genes selected from the group consisting of ATP6V0A1, ARRDC3, CD23, CD200, CD300C, CYSLTR1, ITGB1, MBOAT2, Met, MYO9A, PTPN13, S100P, SECTM1, TCN2, TSPAN2 and VSIG1.

The present invention also provides a method of providing information to predict the tumor reactivity of lymphocytes, which includes measuring an mRNA level or a protein level for ITGA6 gene.

The prediction method may further include measuring mRNA levels or proetein levels for one or more types of genes selected from the group consisting of ATP6V0A1, ARRDC3, CD23, CD200, CD300C, CYSLTR1, ITGB1, MBOAT2, Met, MYO9A, PTPN13, S100P, SECTM1, TCN2, TSPAN2 and VSIG1.

To discover a genetic marker capable of effectively selecting lymphocytes exhibiting tumor-specific activity, through an specific example, the inventors discovered a total of 17 types of genetic markers using lymphocytes isolated from tumor tissues derived from 15 patients with triple-negative breast cancer (TNBC). In more detail, in one embodiment of the present invention, as a result of co-culturing tumor infiltrating lymphocytes (TIL) and breast cancer cells which are derived from 15 triple-negative breast cancer patients, 6 patients were confirmed as showing a reactivity. As a result of analyzing genes differentially expressed in 6 patients showing reactivity and 9 patients not showing reactivity, it was confirmed that the expression of a total of 709 genes was differentially shown, and 17 genes, such as Met, CD200, ITGB1, PTPN13, CYSLTR1, VSIG1, MBOAT2, MYO9A, CD23, S100P, SECTM1, CD300C, TSPAN2, ARRDC3, ITGA6, TCN2 and ATP6V0A1, expressed at a cell surface of the genes were selected (see Example 2).

Further, in another example of the present invention, as a result of analyzing the correlation between the tumor-specific reactivity of TILs derived from TNBC patients and the expression levels of the 17 genes, it was confirmed that the expression level of ITGA6 among these genes is significantly high in TILs derived from patients showing the reactivity for tumor cells, particularly, total TILs, NKT cells and T cells (see Example 3).

The result proved that ITGA6 is a more effective marker gene for predicting the tumor reactivity of patient-derived TILs in breast cancer patients.

The term "prediction of tumor reactivity of lymphocytes" used herein refers to predict whether lymphocytes can induce an anticancer effect on tumors, which is through the induction of an immune response by recognizing and responding to tumor cells as an antigen in a tumor tissue, more preferably an autologous tumor tissue.

In the present invention, the lymphocytes may be isolated from tissue, blood or a body fluid containing tumor tissue, and the body fluid may be ascitic fluid, pleural fluid and bile, which contain lymphocytes, but the present invention is not limited thereto.

The tumor is preferably breast cancer, and more preferably TNBC, but not limited thereto.

In another example, the inventors analyzed the effectiveness of various combination models on the tumor reactivity prediction performance for the differentially expressed 17 genes in Example 2 through machine learning analysis. As a result, the combination model consisting of three genes was confirmed to have the highest prediction accuracy, and specifically, it was confirmed that ATP6V0A1*TSPAN2*MBOAT2 and PTPN13*TCN2*TSPAN2 combinations of the combination variables show significant tumor reactivity prediction performance (see Examples 4 and 5).

In another aspect, the present invention provides a marker composition for predicting the tumor reactivity of lymphocytes, which includes mRNAs or proteins for three or more types of genes selected from the group consisting of ATP6V0A1 (GenBank Accession No.: NM_001130020.3, NM_001130021.3, NM_001378522.1, NM_001378523.1 and NM_001378530.1), MBOAT2 (GenBank Accession No.: NM_001321265.2, NM_001321266.2, NM_001321267.2 and NM_138799.4), PTPN13 (GenBank Accession No.: NM_006264.3, NM_080683.3, NM_080684.3 and NM_080685.2), TCN2 (GenBank Accession No.: NM_000355.4 and NM_001184726.1) and TSPAN2 (GenBank Accession No.: NM_001308315.1, NM_001308316.1 and NM_005725.6).

More preferably, the marker composition includes mRNAs or proteins for ATP6V0A1, MBOAT2 and TSPAN2 genes, or the marker composition includes mRNAs or proteins for PTPN13, TCN2 and TSPAN2 genes, but it is not limited thereto.

The marker composition may also includes mRNA or protein for one or more types of genes selected from the group consisting of ARRDC3 (GenBank Accession No.: NM_001329670.2, NM_001329671.2, NM_001329672.2 and NM_020801.4), CD23 (GenBank Accession No.: NM_001207019.2, NM_001220500.2 and NM_002002.4), CD200 (GenBank Accession No.: NM_001004196.3, NM_001318826.1, NM_001318828.1, NM_001318830.1 and NM_001365851.2), CD300C (GenBank Accession No.: NM_006678.5), CYSLTR1 (GenBank Accession No.: NM_001282186.1, NM_001282187.2, NM_001282188.2 and NM_006639.4), ITGA6 (GenBank Accession No.: NM_000210.4, NM_001079818.3, NM_001316306.2, NM_001365529.2 and NM_001365530.2), ITGB1 (GenBank Accession No.: NM_002211.4, NM_033668.2 and NM_133376.2), Met (GenBank Accession No.: NM_000245.4, NM_001127500.3, NM_001324401.2 and NM_001324402.2), MYO9A (GenBank Accession No.: NM_006901.4), S100P (GenBank Accession No.: NM_005980.3), SECTM1 (GenBank Accession No.: NM_003004.3) and VSIG1 (GenBank Accession No.: NM_001170553.1 and NM_182607.5).

The present invention also provides a composition for predicting the tumor reactivity of lymphocytes, which includes an agent capable of measuring mRNAlevels or an agent capable of measuring protein levels for three or more types of genes selected from the group consisting of ATP6V0A1, MBOAT2, PTPN13, TCN2 and TSPAN2, and a kit for predicting the tumor reactivity of lymphocytes, which includes the composition.

More preferably, the composition includes an agent capable of measuring mRNA levels or an agent capable of measuring protein levels for ATP6V0A1, MBOAT2 and TSPAN2 genes. In addition, the composition includes an agent capable of measuring mRNA levels or an agent capable of measuring of PTPN13, TCN2 and TSPAN2 genes, but not limited thereto.

The composition may also include an agent capable of measuring mRNAlevels or an agent capable of measuring protein levels for one or more types of genes selected from the group consisting of ARRDC3, CD23, CD200, CD300C, CYSLTR1, ITGA6, ITGB1, Met, MYO9A, S100P, SECTM1 and VSIG1.

The present invention also provides a method of providing information to predict tumor reactivity of lymphocytes, which includes measuring mRNA levels or protein levels for three or more types of genes selected from the group consisting of ATP6V0A1, MBOAT2, PTPN13, TCN2 and TSPAN2.

The prediction method may further include measuring mRNA levels or protein levels for one or more types of genes selected from the group consisting of ARRDC3, CD23, CD200, CD300C, CYSLTR1, ITGA6, ITGB1, Met, MYO9A, S100P, SECTM1 and VSIG1.

In the present invention, the agent capable of measuring mRNA level may be sense and anti-sense primers complementarily binding to mRNA of a gene, but it is not limited thereto.

The term "primer" used herein refers to a short gene sequence serving as the initiation site of DNA synthesis, and an oligonucleotide synthesized for the purpose of the use in diagnosis and DNA sequencing. The primers may be conventionally used by being synthesized in a length of 15 to 30 bp, but may vary according to the purpose of use, and the primers may be modified by methylation or capping according to a conventional method.

The term "probe" used herein refers to a nucleic acid capable of specifically binding to mRNA having a length of several to hundreds of bases, produced through enzymatic chemical isolation and purification or synthesis process. The presence or absence of mRNA can be confirmed by labeling a radioactive isotope or enzyme, and the probe may be used by designing and modified according to a known method.

In the present invention, the agent capable of measuring protein level may be an antibody specifically binding to the protein encoded by a gene, but the present invention is not limited thereto.

The term "antibody" used herein includes an immunoglobulin molecule having an specific antigen immunologically, and includes all of monoclonal antibodies and polyclonal antibodies. In addition, the antibody includes all of types produced by genetic engineering, such as chimeric antibodies (e.g., a humanized murine antibody) and heterologous antibodies (e.g., bispecific antibodies).

The kit for predicting the tumor reactivity of lymphocytes according to the present invention consists of a composition, solution or device including one component or more types of different components, which are suitable for an analysis method.

In the method of providing information to predict tumor reactivity of lymphocytes according to the present invention, the lymphocyte may be isolated from tissue, blood or body fluid derived from a subject, but not limited thereto.

The subject is preferably a breast cancer patient, and more preferably a TNBC patient, but not limited thereto.

The "method of providing information to predict tumor reactivity of lymphocytes" used herein is a method of providing basic information required for selection of only lymphocytes exhibiting activity specific for tumor cells as a preliminary step for immunotherapy using lymphocytes.

In the present invention, the mRNA level may be measured by a conventional method known in the art, such as one or more methods selected from the group consisting of polymerase chain reaction (PCR), reverse transcriptase PCR (RT-PCR), real-time PCR, RNase protection assay (RPA), a microarray, and northern blotting, but the present invention is not limited thereto.

In the present invention, the protein level may be measured by a conventional method known in the art, such as one or more methods selected from the group consisting of western blotting, radioimmunoassay (RIA), radioimmunodiffusion, enzyme-linked immunosorbent assay (ELISA), immunoprecipitation, flow cytometry, immunofluorescence, Ouchterlony diffusion, complement fixation assay, and a protein chip, but the present invention is not limited thereto.

Hereinafter, to help in understanding the present invention, exemplary examples will be suggested. However, the following examples are merely provided to more easily understand the present invention, and not to limit the present invention.

### [Examples]

### Example 1. Preparation of experiment and experimental methods

### 1-1. Culture of tumor-infiltrating lymphocytes (TILs)

To isolate and culture tumor-infiltrating lymphocytes (TILs), first, breast cancer tissue collected immediately after surgery was brought into the laboratory, and then TILs were isolated from the tumor tissue within 2 hours, followed by culturing according to the following method. Here, all of the breast cancer tissue used herein was only derived from breasts except breast tissue derived from lymph nodes where breast cancer had metastasized. More specifically, the tumor tissue was washed with phosphate buffered saline (PBS, pH 7.4, Biowest) containing 1X ZellShield antibiotic (Minerva Biolabs, Berlin, Germany), and cut into pieces with a diameter of 1 mm. Afterward, to isolate TILs from the tissue, two pieces were dispensed into each well in a 24-well plate in which 2 mL of RPMI 1640 medium (Life Technologies, NY, USA) containing 10% fetal bovine serum (FBS, Corning, VA, USA), 1× ZellShield, 50 nM 2-mercaptoethanol (Life Technologies, NY, USA) and 1,000 IU/mL human recombinant IL-2 (Miltenyi Biotec, Auburn, CA, USA) was contained per well, and then incubated at 37 °C in a 5% CO₂ incubator for 14 days. Half of the medium was changed every 2 days during the culture period, and the cells were divided into two wells when the color of the medium changed from red to yellow. After 14 days, to remove tumor tissue and residual materials, the cultured TILs were filtered through a nylon mesh strainer with 40-µm pores, and centrifuged at 1,500 rpm for 5 minutes, and the number and survival rate of cells were confirmed, followed by cryopreservation until the next experiment was performed.

Meanwhile, for additional rapid expansion (REP), the TILs isolated by the above-described method were cultured in an REP medium (50% RPMI 1640 and 50% AIM-V medium, Life Technologies) containing 10% FBS, 1 × ZellShield, 1,000 IU/mL human recombinant IL-2, and 30 ng/mL human anti-CD3 antibodies (OKT3, Miltenyi Biotec, Bergisch Gladbach, Germany) along with irradiated (50 Gy) allogeneic peripheral blood mononuclear cells (PBMCs) obtained from healthy donors. The REP medium was newly added every two or three days, after 14 days, cultured TILs (post-REP TILs) were collected and cryopreserved.

### 1-2. Primary cancer cell culture

To isolate and culture primary cancer cells from tumor tissue, the breast cancer tissue section obtained by the method described in Example 1-1 was incubated in a 5% CO₂ incubator at 37 °C for 1 hour to be digested in the presence of a digestion buffer, that is, a DMEM-F12 medium (Life Technologies) containing 2% FBS, 1x penicillin/streptomycin (Invitrogen, CA, USA), 10 µg/mL insulin (Life Technologies), 10 ng/mL EGF (Invitrogen), and 1× collagenase/hyaluronidase (Gendepot, Barker, TX, USA). Afterward, the pellet obtained from the digested tissue was centrifuged for 30 seconds at 80 x g, resuspended in 0.25% trypsin/EDTA and dissociated into single cells by pipetting. The suspension containing single cells was filtered through a 100-µm pore strainer, and then the cells were washed with a cold Hank's balanced salt solution (HBSS) containing 2% FBS, and centrifuged for 5 minutes at 300 x g, thereby obtaining single cancer cells. The dissociated cells obtained by the above-described method were cultured at 37 °C in a CO₂ incubator, on a 100 mm collagen I-coated plate (Corning) using a DMEM/F12 (1:1) medium containing 2% FBS, 5 ng/mL human recombinant EGF, 0.3 µg/mL hydrocortisone (Sigma-Aldrich, St. Louis), 0.5 ng/mL cholera toxin (Sigma-Aldrich), 5nM 3,3', 5-triiodo-L-thyronine (Sigma-Aldrich), 0.5 nM β-estradiol (Sigma-Aldrich), 5 µM isoproterenol hydrochloride (Sigma-Aldrich), 50 nM ethanolamine (Sigma-Aldrich), 50 nM O-phosphorylethanolamine (Sigma-Aldrich), 1 × insulin/transferrin/selenium, and 1% penicillin/streptomycin (Life Technologies). Afterward, the cells were sub-cultured at least twice before cryopreservation.

### 1-3. Evaluation of reactivity of TILs

To evaluate the potential functionality of TILs proliferated on a large scale by the method described in Example 1-1, 1 x 10⁵ TILs per well were stimulated with 32.4 nM PMA and 1 µg/mL ionomycin in a 96-well plate for 24 hours. Subsequently, the culture plate was centrifuged at 1,500 rpm for 5 minutes to collect a supernatant, followed by measuring an IFN-γ protein level through ELISA.

In addition, to investigate the reactivity of TIL for autologous cancer cells, 4 x 10⁵ TILs were co-cultured with 1 x 10⁵ autologous breast cancer cells on a 96-well plate for 24 hours, a supernatant was collected, and then a level of IFN-γ protein secreted from TILs was measured through ELISA.

### 1-4. ELISA

In this example, ELISA was performed according to the manufacturer's protocol using an ELISA kit (K0331121, Koma Biotech, Seoul, Korea). Briefly, each well was washed with a washing solution, a sample, a standard material and a blank were added to respective wells and incubated for 2 hours at room temperature, and all tests were performed two or three times. Subsequently, after removing the liquid, the plate was washed with a washing solution, and incubated at room temperature for 2 hours by adding a biotinylated detection antibody. Afterward, the plate was washed again, and a streptavidin-horseradish peroxidase conjugate was added and then cultured at 37 °C for 30 minutes. After washing, a 3, 3', 5, 5'-tetramethylbenzidine solution was added, and cultured at room temperature for suitable color development. A stop solution was added to each well, and IFN-γ levels were measured at 450 nm using a microplate reader (Spectramax 340PC, Molecular Devices).

### 1-5. Statistical analysis

To discover a genetic marker capable of distinguishing lymphocytes having activity specific for autologous tumor cells, statistical analyses were performed by the following method:
1) A ratio was obtained by dividing an IFN-γ level secreted from TILs when cultured together with autologous tumor cells according to the method described in Example 1-3 by an IFN-γ level secreted when TILs were present alone, and when the value was 2 or more, it was defined as reactive.
2) The differentially expressed genes were analyzed by extracting RNA from the cultured TILs of a group with reactivity to tumors shown by the 1) analysis and a non-reactive group and performing transcriptome sequencing. A gene with a more than two-fold difference in expression level was defined as differentially expressed.
3) A ratio obtained by dividing an IFN-γ level secreted from TILs in co-culture with tumor cells by an IFN-γ level secreted when TILs were present alone and an expression level of each gene were investigated through bivariate correlation analysis (Spearman Correlation), thereby selecting a gene group showing a significant correlation (p < 0.05).

### Example 2. Search for tumor-specific lymphocyte selection marker

As a result of culturing TILs derived from 15 triple-negative breast cancer (TNBC) patients along with breast cancer cells derived from each patient according to the method described in Example 1-3, reactivity was shown in 6 patients. Subsequently, as a result of analyzing genes differentially expressed in the group of six patients showing reactivity and the group of 9 patients not showing reactivity according to the second method described in Example 1-5, it was confirmed that, among 13,827 genes, a total of 709 gene expressions were differentially shown, and among these genes, 17 genes expressed at the cell surface are shown in Table 1 below. Based on the genes expressed at the cell surface, live cells may be selected through a method such as FACS, etc.

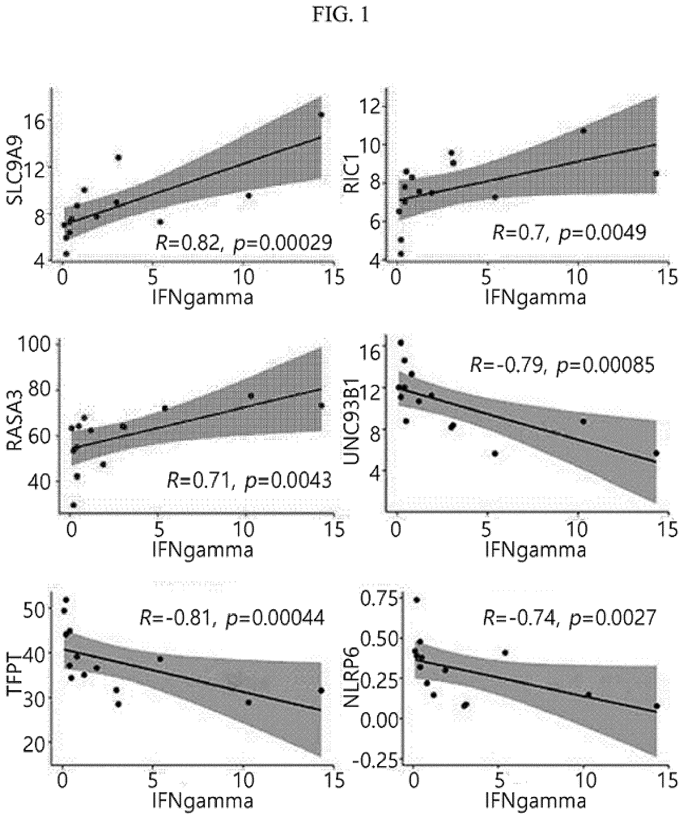

Further, as seen from the result of FIG. 1, the total number of genes showing a significant relationship with an IFN-γ ratio through the correlation analysis disclosed in the third method described in Example 1-5 is 1,923, and among the differentially expressed 709 genes, it was confirmed that there are a total of 46 genes in common. Among the 17 genes in Table 1 below, it was confirmed that genes included in the 46 genes are the 8 genes present at the top.

**[Table 1]**

| **Gene** | **Official full name** |
|---|---|
| **Met** | MET proto-oncogene, receptor tyrosine kinase |
| **CD200** | CD200 molecule |
| **ITGB1 (CD29)** | integrin subunit beta 1 |
| **PTPN13** | protein tyrosine phosphatase non-receptor type 13 |
| **CYSLTR1** | cysteinyl leukotriene receptor 1 |
| **VSIG1** | V-set and immunoglobulin domain containing 1 |
| **MBOAT2** | membrane bound O-acyltransferase domain containing 2 |
| **MYO9A** | myosin IXA |
| CD23 | Fc fragment of IgE receptor II; FCER2 |
| S100P | S100 calcium binding protein P |
| SECTM1 | secreted and transmembrane 1 |
| CD300C | CD300c molecule |
| TSPAN2 | tetraspanin 2 |
| ARRDC3 | arrestin domain containing 3 |
| ITGA6 (CD49f) | integrin subunit alpha 6 |
| TCN2 | transcobalamin 2 |
| ATP6V0A1 | ATPase H+ transporting V0 subunit a1 |

### Example 3. Verification of effectiveness of marker gene for predicting tumor reactivity

### 3-1. Analysis of correlation between tumor-specific reactivity of TNBC-REP TILs and expression levels of 17 types of genes

The inventors conducted the following experiment to verify the effectiveness as a marker capable of selecting tumor-specific lymphocytes for the 17 genetic markers obtained in Example 2.

First, the expression levels of the 17 genes obtained in Example 2 in TILs which were isolated from 14 TNBC patients and rapidly expanded (TNBC-REP TILs) were measured using FACS, and the result is shown in Table 2 below.

**[Table 2]**

| **Sa mpl e:** | **Rea ctivi ty** | **CD 23** | **ITG B1** | **ITG A6** | **CD 200** | **CD 300 c** | **Met** | **S10 0P** | **TSP AN** | **AR RD C3** | **AT P6 VO A** | **CY S LT R1** | **MB OA T2** | **MY O9 A** | **PT PN1 3** | **SE C TM 1** | **TC N2** | **VSI G1** |
|---|---|---|---|---|---|---|---|---|---|---|---|---|---|---|---|---|---|---|
| BC1 544 | 0 | 0.1 | 100. 0 | 7.0 | 0.3 | 46.8 | 0.1 | 0.1 | 0.0 | 0.1 | 0.3 | 0.8 | 0.3 | 0.0 | 0.1 | 0.6 | 0.1 | 0.6 |
| BC1 604 8 | 0 | 0.3 | 100. 0 | 4.1 | 1.9 | 67.1 | 0.1 | 0.0 | 0.0 | 0.2 | 0.2 | 0.9 | 0.6 | 0.1 | 0.1 | 0.9 | 0.1 | 0.5 |
| BC1 609 2 | 1 | 0.0 | 100. 0 | 33.9 | 6.9 | 68.4 | 0.1 | 0.0 | 0.0 | 0.1 | 0.1 | 0.2 | 0.2 | 0.1 | 0.0 | 0.5 | 0.1 | 0.1 |
| BC1 612 6 | 0 | 0.1 | 99.9 | 5.5 | 0.3 | 40.2 | 0.2 | 0.1 | 0.0 | 0.2 | 0.3 | 0.6 | 0.4 | 0.0 | 0.1 | 0.6 | 0.1 | 0.1 |
| BC1 612 7 | 1 | 0.2 | 99.8 | 80.9 | 0.5 | 34.7 | 0.1 | 0.0 | 0.0 | 0.1 | 0.1 | 0.1 | 0.1 | 0.0 | 0.1 | 0.3 | 0.0 | 0.0 |
| BC1 614 3 | 0 | 0.8 | 99.9 | 59.8 | 0.6 | 14.4 | 0.2 | 0.0 | 0.0 | 0.1 | 0.2 | 0.3 | 0.1 | 0.1 | 0.1 | 0.1 | 0.1 | 0.4 |
| BC1 614 7 | 1 | 0.6 | 99.6 | 43.9 | 1.1 | 23.6 | 0.2 | 0.0 | 0.0 | 0.1 | 0.3 | 0.2 | 0.2 | 0.0 | 0.0 | 0.5 | 0.1 | 0.2 |
| BC1 615 1 | 0 | 0.1 | 99.6 | 31.5 | 0.3 | 34.2 | 0.2 | 0.1 | 0.0 | 0.1 | 0.2 | 0.4 | 0.4 | 0.1 | 0.0 | 0.3 | 0.2 | 0.2 |
| BC1 615 4 | 1 | 0.1 | 100. 0 | 81.0 | 3.5 | 93.8 | 0.1 | 0.0 | 0.0 | 0.0 | 0.6 | 0.1 | 0.2 | 0.0 | 0.0 | 0.1 | 0.1 | 0.0 |
| BC1 615 8 | 1 | 5.4 | 99.0 | 68.2 | 4.3 | 49.1 | 0.1 | 0.0 | 0.0 | 0.0 | 0.1 | 0.1 | 0.1 | 0.0 | 0.0 | 0.2 | 0.0 | 0.1 |
| BC1 616 6 | 0 | 0.2 | 100. 0 | 22.5 | 1.2 | 9.5 | 0.2 | 0.1 | 0.0 | 0.1 | 0.3 | 0.1 | 0.4 | 0.1 | 0.0 | 0.5 | 0.1 | 0.1 |
| BC1 620 8 | 0 | 0.5 | 99.7 | 14.7 | 0.7 | 57.3 | 0.2 | 0.1 | 0.0 | 0.2 | 0.2 | 0.2 | 0.1 | 0.0 | 0.0 | 0.2 | 0.1 | 0.2 |
| BC1 622 3 | 0 | 0.0 | 99.8 | 7.1 | 0.3 | 19.8 | 0.3 | 0.1 | 0.0 | 0.1 | 0.3 | 0.9 | 0.8 | 0.1 | 0.1 | 0.4 | 0.1 | 0.3 |
| BC1 700 9 | 0 | 0.0 | 99.8 | 38.4 | 0.2 | 56.2 | 0.3 | 0.0 | 0.0 | 0.2 | 0.3 | 0.2 | 0.2 | 0.0 | 0.1 | 0.5 | 0.1 | 0.0 |

Further, to investigate whether there is a significant difference between expression levels of genes expressed in reactive TILs (reactivity=1) and non-reactive TILs (reactivity=0), SPSS statistical analysis was performed. As a result, as can be seen in Table 3 below, it was confirmed that there is a significant difference between ITGA6, CD200, S100P, ARRDC3, CYSLTR1 and VSIG1 among the 17 genes.

**[Table 3]**

| | **CD23** | **ITGB1** | **ITGA6** | **CD200** | **CD300 c** | **Met** | **S100P** | **TSPA N** | **ARRD C3** |
|---|---|---|---|---|---|---|---|---|---|
| **U of** | 18.000 | 18.500 | 3.000 | 6.000 | 14.000 | 8.500 | 7.500 | 22.500 | .000 |
| **Mann-Whitney** | | | | | | | | | |
| **W of Wilcoxon** | 63.000 | 33.500 | 48.000 | 51.000 | 59.000 | 23.500 | 22.500 | 37.500 | 15.000 |
| **Z** | -.610 | -.549 | -2.600 | -2.225 | -1.133 | -2.032 | -2.327 | .000 | -3.003 |
| **Approximate significance probability (two-tailed)** | .542 | .583 | .009 | .026 | .257 | .042 | .020 | 1.000 | .003 |
| **Exact significance probability [2* (one-tailed significance probability)]** | .606^{b} | .606^{b} | .007^{b} | .029^{b} | .298^{b} | .060^{b} | .042^{b} | 1.000^{b} | .001^{b} |
| **Exact significance probability (two-tailed)** | .571 | .610 | .007 | .025 | .298 | .073 | .031 | 1.000 | .001 |
| **Exact significance probability (one-tailed)** | .290 | .327 | .003 | .013 | .149 | .048 | .028 | 1.000 | .000 |
| **Point probability** | .019 | .045 | .001 | .004 | .029 | .045 | .028 | 1.000 | .000 |

| | **ATP6 VOA** | **CYS LTR1** | **MBOA T2** | **MYO9 A** | **PTPN1 3** | **SEC TM1** | **TCN2** | **VSIG 1** | |
|---|---|---|---|---|---|---|---|---|---|
| **U of Mann-Whitney** | 15.500 | 6.000 | 10.500 | 20.500 | 11.000 | 14.500 | 14.500 | 7.500 | |
| **W of Wilcoxon** | 30.500 | 21.000 | 25.500 | 35.500 | 26.000 | 29.500 | 29.500 | 22.500 | |
| **Z** | -.944 | -2.202 | -1.605 | -.267 | -1.533 | -1.068 | -1.069 | -2.002 | |
| **Approximate significance probability (two-tailed)** | .345 | .028 | .108 | .789 | .125 | .286 | .285 | .045 | |
| **Exact significance probability [2* (one-tailed significance probability)]** | .364^{b} | .029^{b} | .112^{b} | .797^{b} | .147^{b} | .298^{b} | .298^{b} | .042^{b} | |
| **Exact significance probability (two-tailed)** | .375 | .027 | .118 | .823 | .147 | .313 | .315 | .045 | |
| **Exact significance probability (one-tailed)** | .188 | .013 | .059 | .411 | .073 | .156 | .159 | .022 | |
| **Point probability** | .018 | .002 | .008 | .025 | .017 | .014 | .020 | .003 | |

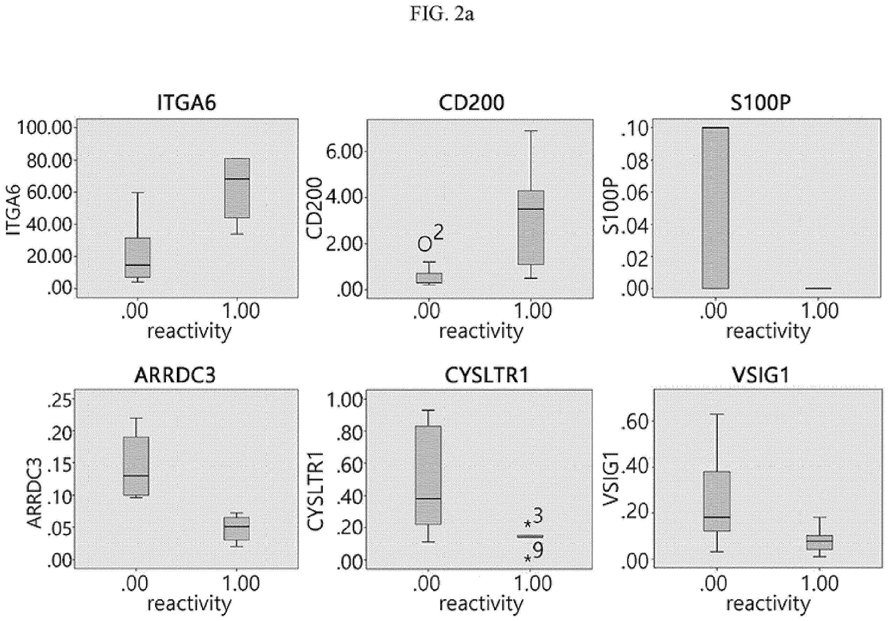

Specifically, as a result of quantitatively graphing the difference in expression level according to reactivity for each of the 6 genes, as shown in FIG. 2a, the expression levels of ITGA6 and CD200 are higher in TILs (1.00) of a reactive group, compared to a non-reactive group (00), and the expression levels of S100P, ARRDC3, CYSLTR1 and VSIG1 are lower in TILs of the reactive group. Accordingly, from the above results, as a marker for selecting reactive TILs, ITGA6 and CD200 having increased expression levels were selected and subjected to the following experiment.

### 3-2. Analysis of expression levels of ITGA6 and CD200 per TIL type

The inventors attempted to examine whether, in which cells among tumor-infiltrating immune cells (TILs), the expression of ITGA6 and CD200 selected in Example 3-1 has a difference. To this end, after FACS analysis was performed by staining TILs with CD3, CD4, CD8 or CD56 antibodies and ITGA6 or CD200, ITGA6 and CD200 expression was analyzed, in each of total TILs (CD45+), NKT cells (CD45+ CD56+ CD3+), T cells (CD45+ CD56- CD3+), CD4 T cells (CD45+ CD56-CD3+ CD4+ CD8-) and CD8 T cells (CD45+ CD56- CD3+ CD4- CD8+), and the result is shown in Tables 4 and 5 below.

**[Table 4]**

| **Sample** | **ITGA6+ CD45** | **ITGA6+ NKT cells** | **ITGA6+ T cells** | **ITGA6+ CD4 T cells** | **ITGA6+ CD8 T cells** |
|---|---|---|---|---|---|
| BC1544 | 15.9 | 2.47 | 12.7 | 21.6 | 3.09 |
| BC16048 | 11.7 | 4.65 | 11.4 | 30.5 | 4.35 |
| BC16092 | 47 | 59.6 | 43.9 | 50 | 48 |
| BC16126 | 10.6 | 13.1 | 8.76 | 48.9 | 10.5 |
| BC16127 | 88.5 | 77.3 | 86.5 | 96 | 77.5 |
| BC16143 | 77.3 | 72.7 | 72.6 | 74 | 59 |
| BC16147 | 55.4 | 26.5 | 50.7 | 63.7 | 41.7 |
| BC16151 | 41.6 | 24.6 | 37.7 | 76.2 | 40.5 |
| BC16154 | 89 | 50 | 87.5 | 89.5 | 41.6 |
| BC16158 | 78.9 | 72.2 | 75.7 | 85.3 | 38.6 |
| BC16166 | 31.3 | 22.8 | 27.5 | 65.6 | 19.6 |
| BC16208 | 13.7 | 12.2 | 11.4 | 41.8 | 13.8 |
| BC16223 | 24.8 | 9.69 | 21.7 | 28.2 | 14.6 |
| BC17009 | 53.8 | 25.7 | 54.2 | 92.7 | 52.2 |

**[Table 5]**

| **Sample** | **CD200+ CD45** | **CD200+ NKT cells** | **CD200+ T cells** | **CD200+ CD4 T cells** | **CD200+ CD8 T cells** |
|---|---|---|---|---|---|
| BC1544 | 0.5 | 1.33 | 0.26 | 0.34 | 0.2 |
| BC16048 | 4.33 | 3 | 2.84 | 7.24 | 0.73 |
| BC16092 | 9.45 | 15.1 | 6.84 | 3.66 | 8.59 |
| BC16126 | 0.33 | 0.85 | 0.22 | 0.49 | 0.1 |
| BC16127 | 0.42 | 0.92 | 0.14 | 0.33 | 0.061 |
| BC16143 | 0.98 | 25 | 0.52 | 0.61 | 0.43 |
| BC16147 | 1.38 | 5.36 | 0.81 | 1.68 | 0.074 |
| BC16151 | 0.23 | 1.89 | 0.11 | 0.66 | 0.11 |
| BC16154 | 6.45 | 4.35 | 3.8 | 4.25 | 4.12 |
| BC16158 | 9.22 | 21.4 | 6.1 | 8.07 | 0 |
| BC16166 | 1.9 | 10.1 | 1.12 | 5.57 | 0.036 |
| BC16208 | 1.12 | 3.59 | 0.7 | 1.02 | 0.12 |
| BC16223 | 1.8 | 1.75 | 1.31 | 4.03 | 0.51 |
| BC17009 | 1.15 | 6.29 | 0.28 | 1.44 | 0.19 |

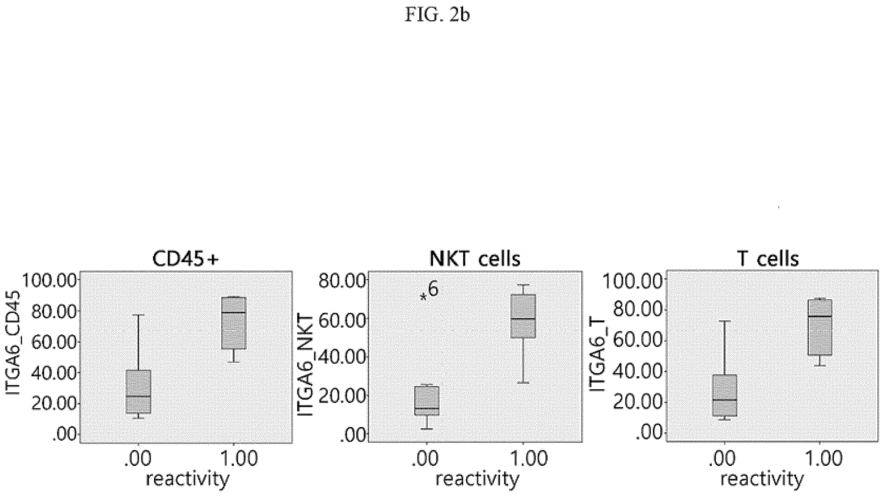

Further, as a result of statistical analysis using an SPSS program, as shown in Table 6 below and FIG. 2b, it was confirmed that ITGA6 expression is significantly higher in a reactive group, compared to a group with no reactivity in total TILs (CD45+), NKT cells and T cells. However, CD200 did not show a significant difference in expression in NKT cells and T cells as well as total TILs. From this result, CD200 was determined as an experimental variable, and ITGA6 was selected as a reactivity prediction marker for tumor-specific lymphocytes.

**[Table 6]**

| | **CD20 0_ CD45** | **CD20 0_ NKT** | **CD20 0 T** | **CD20 0_ CD4 T** | **CD20 0_ CD8 T** | **ITGA 6_ CD45** | **ITGA 6_ NKT** | **ITG A6_ T** | **ITGA 6_ CD4T** | **ITGA 6_ CD8T** |
|---|---|---|---|---|---|---|---|---|---|---|
| **U of Mann-Whitney** | 10.00 0 | 16.00 0 | 11.00 0 | 17.00 0 | 20.00 0 | 3.000 | 4.000 | 4.000 | 10.000 | 9.000 |
| **W of Wilcoxon** | 55.00 0 | 61.00 0 | 56.00 0 | 62.00 0 | 35.00 0 | 48.00 0 | 49.00 0 | 49.00 0 | 55.000 | 54.000 |
| **Z** | -1.667 | -.867 | -1.533 | -.733 | -.333 | -2.600 | -2.467 | 2.469 | -1.667 | -1.800 |
| **Approximate significance probability (two-tailed)** | .096 | .386 | .125 | .463 | .739 | .009 | .014 | .014 | .096 | .072 |
| **Exact significance probability [2* (one-tailed significance probability)]** | .112^{b} | .438^{b} | .147^{b} | .518^{b} | .797^{b} | .007^{b} | .012^{b} | .012^{b} | .112^{b} | .083^{b} |
| **Exact significance probability (two-tailed)** | .112 | .438 | .147 | .518 | .797 | .007 | .012 | .011 | .112 | .083 |
| **Exact significance probability (one-tailed)** | .056 | .219 | .073 | .259 | .399 | .003 | .006 | .006 | .056 | .041 |
| **Point probability** | .014 | .037 | .017 | .040 | .049 | .001 | .002 | .002 | .014 | .011 |

### Example 4. Verification of effectiveness of marker gene for predicting tumor reactivity by machine learning

The inventors conducted analysis for obtaining a biomarker gene capable of predicting reactivity and non-reactivity to tumors by machine learning using a tumor size and the presence or absence of neoadjuvant chemotherapy (NAC) from the 17 marker genes obtained in Example 2. To this end, the analysis was carried out using logistic regression among various machine learning methods.

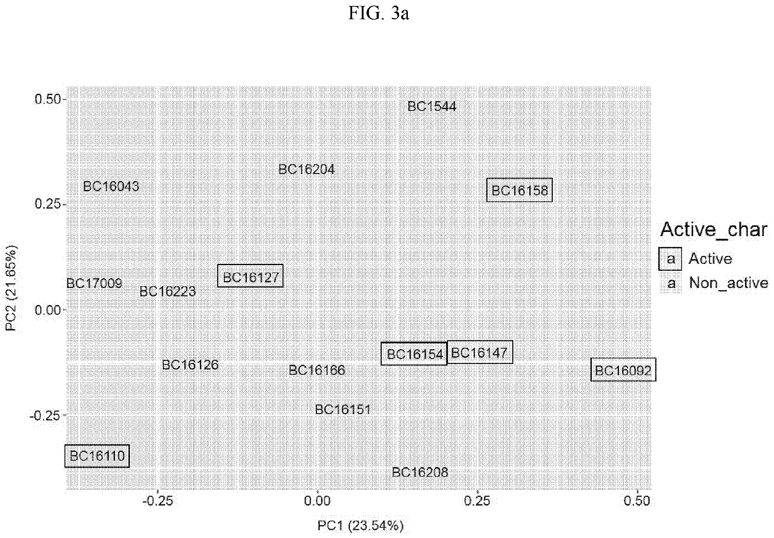

First, principal component analysis (PCA) on TILs derived from 15 TNBC patients was carried out. As a result, as shown in FIG. 3a, although not clearly divided into two clusters, such as a reactive group (Active) and a non-reactive group (Non-active) depending on reactivity, several clusters were observed.

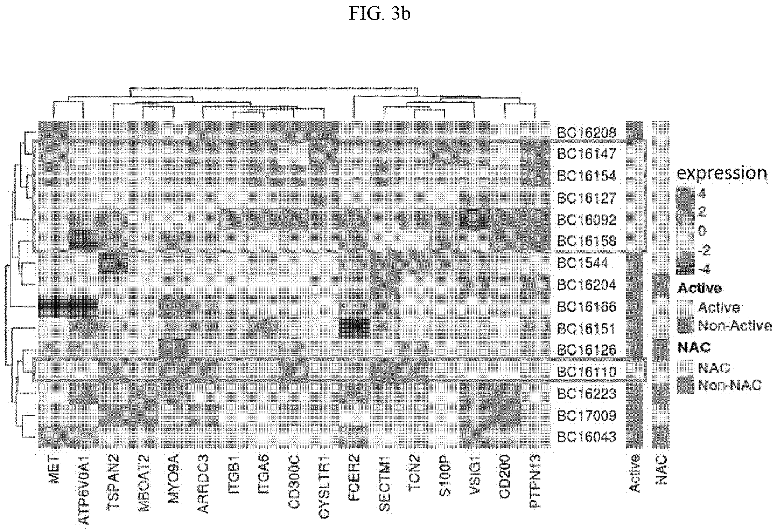

Afterward, the expression levels of 17 types of genes in each patient-derived TILs were analyzed using a heatmap, and the result of a sample derived from a patient showing reactivity was represented in green. As a result of the analysis, as shown in FIG. 3b, among 17 genes, PTPN13 gene showed a high expression level mainly in the reactive group, the MET gene showed a low expression level in a sample subjected to NAC.

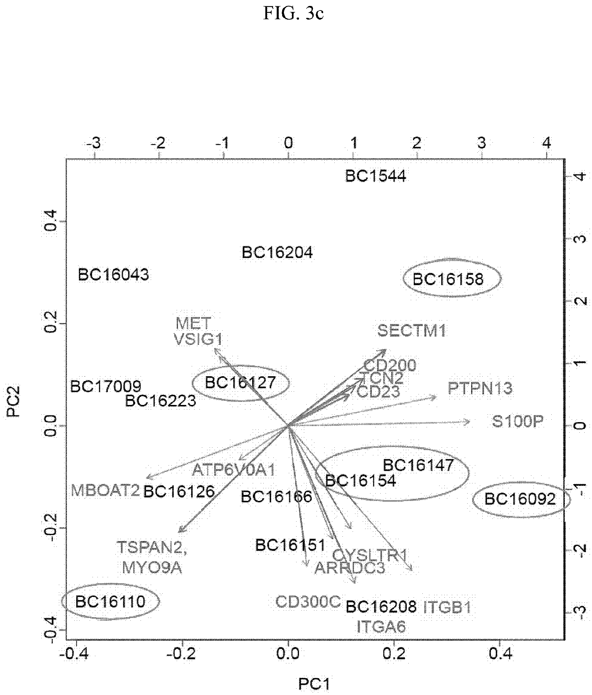

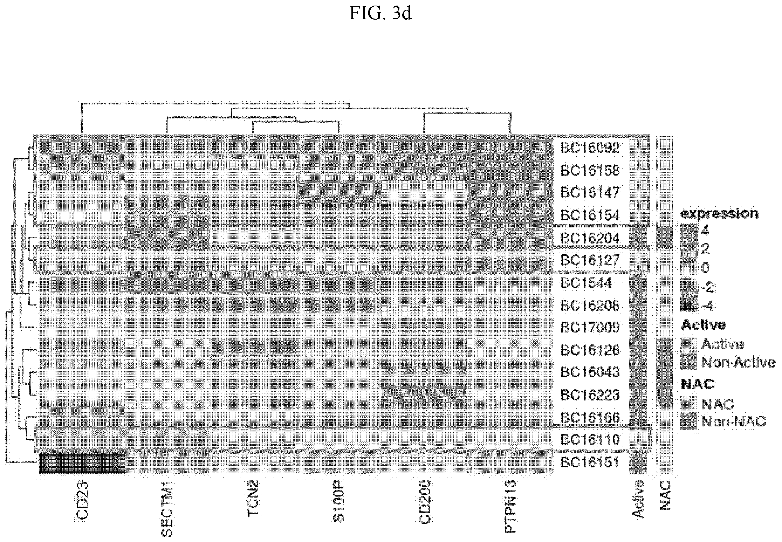

In addition, as a result of analyzing which genes affect each group by displaying the samples and genes together by presenting the PCA analysis result of FIG. 3a in a Biplot graph, as shown in FIG. 3c, it was confirmed that SECTM1, PTPN13, S100P, CD200, TCN2 and FCER2 (CD23) genes are associated with the reactive group excluding a BC16110 sample. Further, as a result of the analysis of the 6 types of genes using a heatmap, as shown in FIG. 3d, the expression level of the PTPN13 gene was generally high in the reactive sample, and it was confirmed that other genes except PTPN13 show no significant difference in expression level.

### Example 5. Discovery of combination of marker genes for predicting tumor reactivity and verification of effectiveness thereof

### 5-1. Discovery and analysis of combination consisting of 10 genes

The inventors conducted analysis for discovering an effective marker combination for selecting lymphocytes having tumor-specific reactivity with respect to the 17 genes obtained in Example 2.

More specifically, for machine learning in consideration of the interactions between 17 types of differentially expressed genes, first, the interaction between the genes was obtained using the PolynomialFeatures library provided by Sklearn. As a result, since there were too many features, lasso was used to select features, and the result is shown in Table 7 below.

**[Table 7]**

| **Feature interaction** | **Number of Features** |
|---|---|
| 2 | 136 -> 10 |
| 3 | 680 -> 11 |
| 4 | 2380 -> 11 |
| 5 | 6188 -> 10 |
| 6 | 12376 -> 8 |
| 7 | 19448 -> 10 |
| 8 | 24310 -> 12 |
| 9 | 24310 -> 11 |
| 10 | 19448 -> 13 |
| 11 | 12376 -> 11 |
| 12 | 6188 -> 7 |
| 13 | 2380 -> 12 |
| 14 | 680 -> 5 |
| 15 | 136 -> 6 |
| 16 | 17 |
| 17 | 1 |

Afterward, oversampling was performed for each interaction using a SMOTE method. Logistic regression was then performed, and as shown in Table 8 below, 10 genes having the highest accuracy and AUC values, except the cases in which the accuracy and AUC values are 1, were selected and their expression levels were compared using a heatmap.

**[Table 8]**

| | **Training Accuracy** | **Test Accuracy** | **AUC** |
|---|---|---|---|
| 2 | 1 | 1 | 1 |
| 3 | 1 | 1 | 1 |
| 4 | 1 | 1 | 1 |
| 5 | 1 | 1 | 1 |
| 6 | 1 | 0.83 | 1 |
| 7 | 1 | 0.83 | 1 |
| 8 | 1 | 0.5 | 0.67 |
| 9 | 1 | 0.5 | 0.78 |
| **10** | **1** | **0.83** | **0.78** |
| 11 | 1 | 0.66 | 0.67 |
| 12 | 1 | 0.5 | 0.11 |
| 13 | 1 | 0.33 | 0.56 |
| 14 | 1 | 0.33 | 0.11 |
| 15 | 1 | 0.33 | 0.11 |
| 16 | 1 | 0.5 | 0.33 |
| 17 | 0.75 | 0.5 | 0.89 |

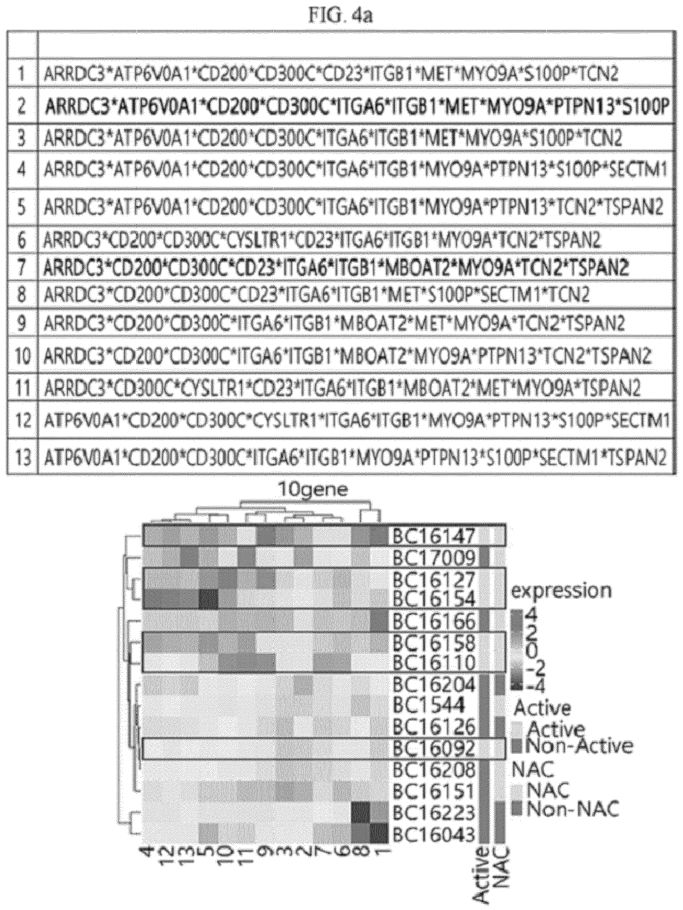

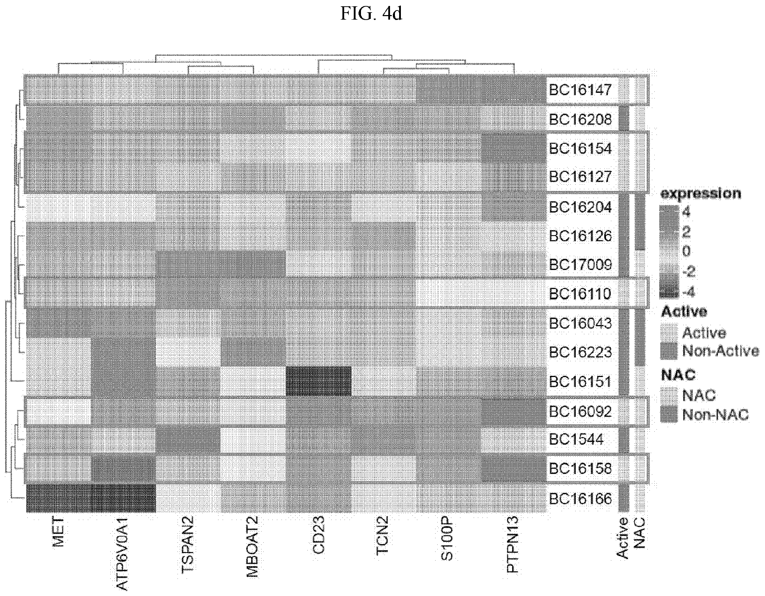

More specifically, in FIG. 4a, the expression level of 10-gene combinations corresponding to 13 features and each gene combination in patient-derived TIL samples was shown. As a result of the analysis, the expression levels of Combinations 4, 12, 13, 5 and 10 from the left became lower in the patient-derived samples showing reactivity. Further, since a positive coefficient value greatly affects prediction as reactive, and a negative coefficient value greatly affects prediction as non-reactive, heatmaps were plotted with each gene combinations 7 (when the coefficient value is positive, red) and 2 (when the coefficient value is negative, green) in which the positive and negative coefficient values are the highest features and analyzed.

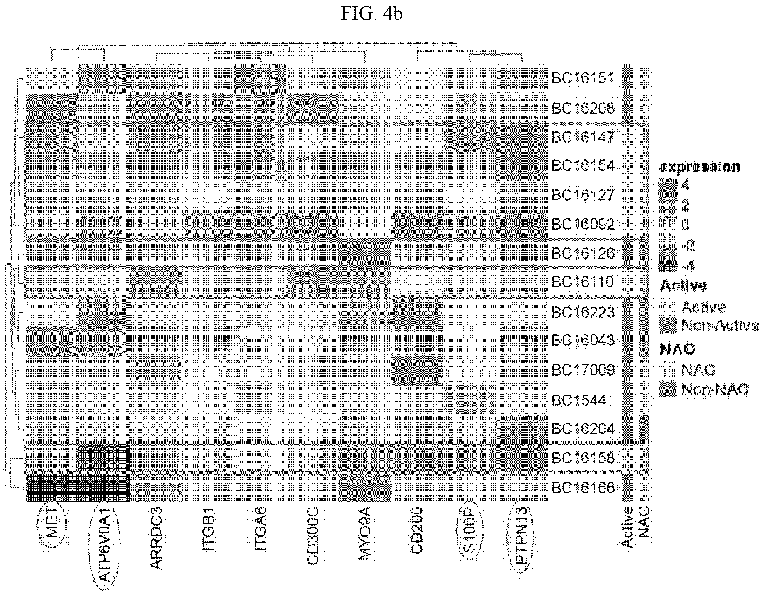

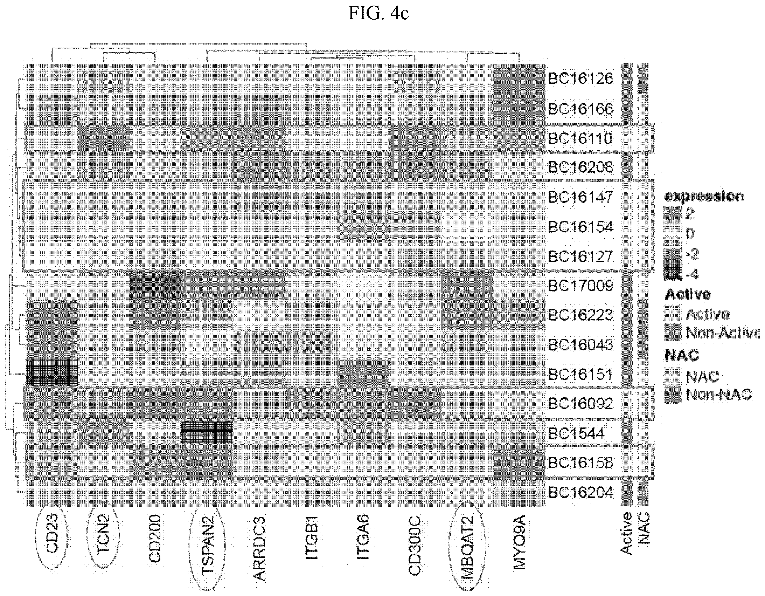

More specifically, a result of the heatmap analysis using 10 genes corresponding to Combination 2 in which the coefficient value is negative is shown in FIG. 4b, and the coefficient value of the combination was -9.30E-05. Compared with the 10 genes of Combination 7 in which the coefficient value is the largest positive number, 4 genes which are not in common were marked with green circles. Among these genes, MET and ATP6V0A1 genes showed low expression levels when NAC was performed, and PTPN13 gene showed a high expression level mainly in a reactive group. In addition, a result of the heatmap analysis using 10 genes corresponding to Combination 7 in which the coefficient value is positive is shown in FIG. 4c, and the coefficient value of the corresponding 10-gene combination was 8.92E-04. The other 4 genes which were not included in the 10 genes of Combination 2 were marked with green circles, and as a result of the analysis of the heatmap result, TSPAN2 and MYO9A genes among the four genes showed low expression levels in the sample subjected to NAC, and the CD300C gene showed a high expression level when NAC was performed. Collectively, there was no difference in expression level according to reactivity of a patient-derived sample between genes from the above results. Therefore, the inventors analyzed a heatmap plotted with a total of 8 genes, which are not in common in Combinations 2 and 7 respectively having negative and positive coefficient values and again confirmed that there was no difference in expression level of genes according to the presence or absence of reactivity. Therefore, the inventors obtained interactions between the 8 genes, such as MET, ATP6V0A1, S100P, PTPN13, CD23, TCN2, TSPAN2 and MBOAT2, and analyzed them by machine learning.

### 5-2. Analysis of combination consisting of 8 genes through machine learning

The inventors conducted an experiment to discover an effective marker combination capable of predicting tumor reactivity using the 8 genes derived in Example 5-1. In consideration of the interactions between the 8 genes by the same method as in Example 5-1, the interactions between genes were obtained using the PolynomialFeatures library, features were selected using lasso, and the result is shown in Table 9 below.

**[Table 9]**

| Feature interaction | Number of Features |
|---|---|
| 2 | 28 -> 15 |
| 3 | 56 -> 20 |
| 4 | 70 -> 11 |
| 5 | 56 -> 10 |
| 6 | 28 -> 10 |
| 7 | 8 |
| 8 | 1 |

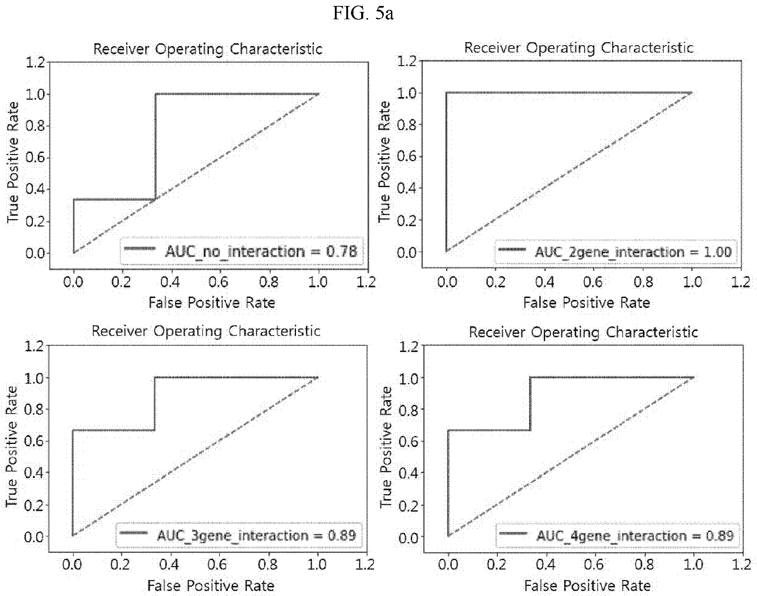

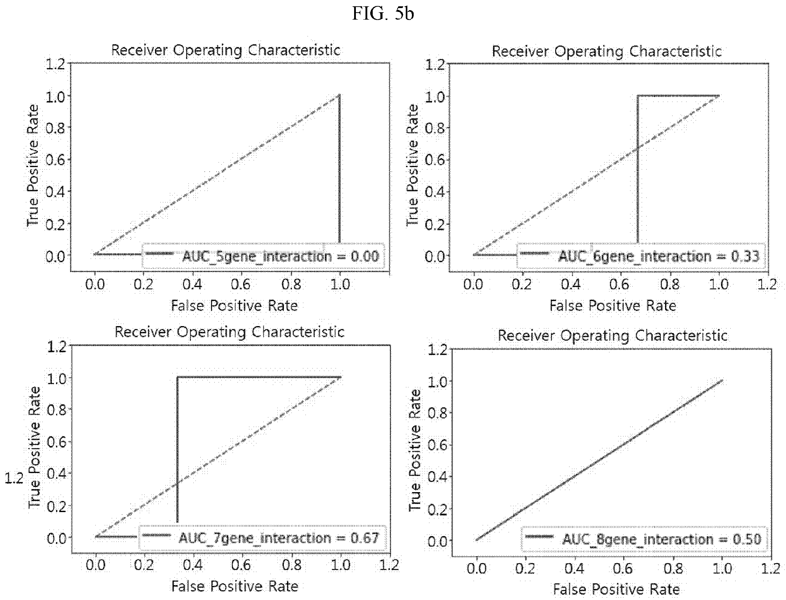

Subsequently, after oversampling was performed to investigate the performance of a model using each gene combination, logistic regression and random forest analysis were carried out. First, as a result of the logistic regression, as shown in Table 10 below, and FIGS. 5A and 5B, when the number of interactions is 3 or 4, except the case in which the test accuracy and AUC value are 1, the test accuracy and AUC value are the highest. In addition, as a result of random forest analysis, as shown in Table 11 below, when the number of interactions is 3, except the case in which the performance of a model is 1, the performance is the highest.

**[Table 10]**

| | **Training Accuracy** | **Test Accuracy** | **AUC** |
|---|---|---|---|
| No-interaction | 1 | 0.66 | 0.78 |
| 2 | 1 | 1 | 1 |
| **3** | **1** | **0.83** | **0.89** |
| **4** | **1** | **0.83** | **0.89** |
| 5 | 1 | 0.5 | 0 |
| 6 | 1 | 0.16 | 0.33 |
| 7 | 1 | 0.33 | 0.67 |
| 8 | 0.5 | 0.5 | 0.5 |

**[Table 11]**

| | **Training Accuracy** | **Test Accuracy** | **AUC** |
|---|---|---|---|
| No-interaction | 1 | 0.83 | 1 |
| 2 | 1 | 0.83 | 1 |
| **3** | **1** | **0.83** | **0.78** |
| 4 | 0.91 | 1 | 1 |
| 5 | 1 | 0.5 | 0.78 |
| 6 | 0.83 | 1 | 1 |
| 7 | 0.91 | 0.5 | 0.67 |
| 8 | 0.83 | 0.33 | 0.28 |

### 5-3. Final selection of combination of marker genes for predicting tumor reactivity

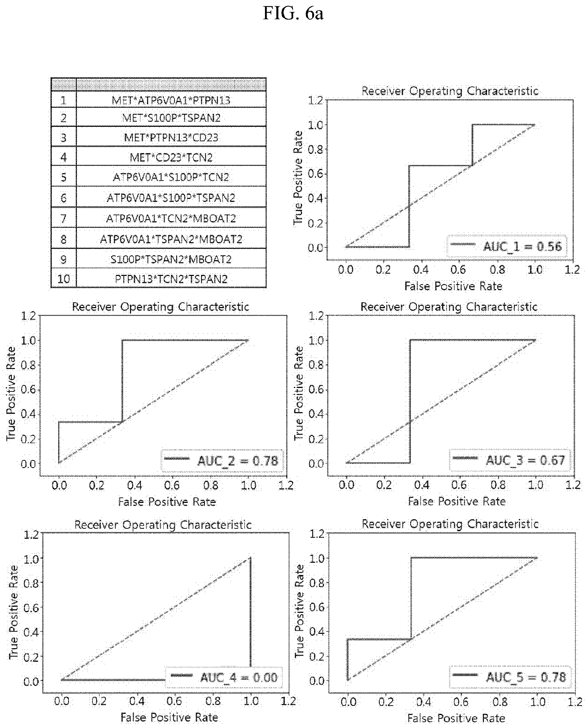

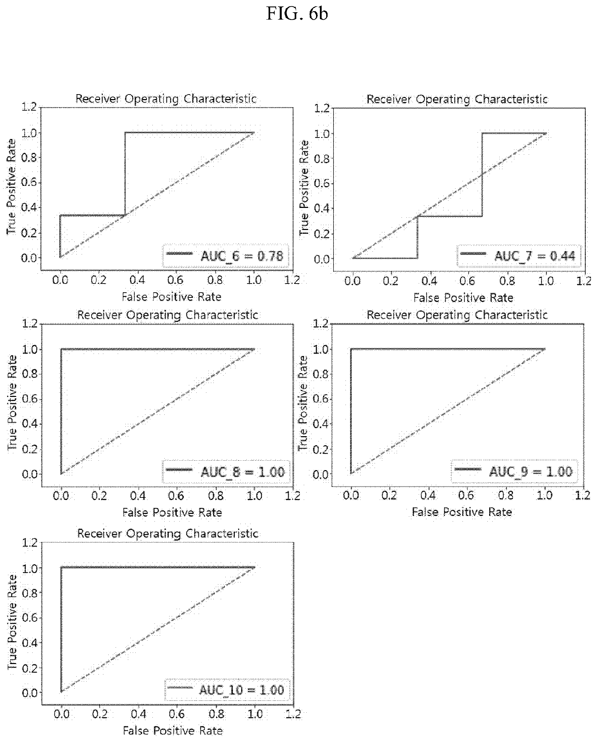

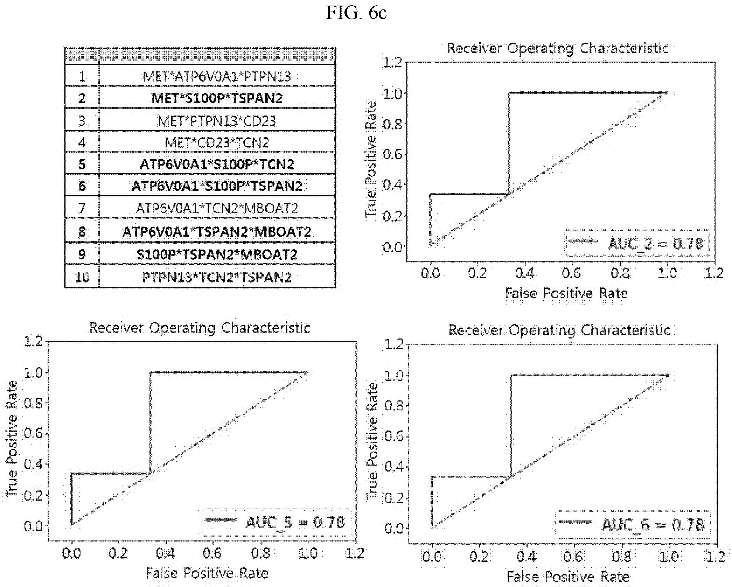

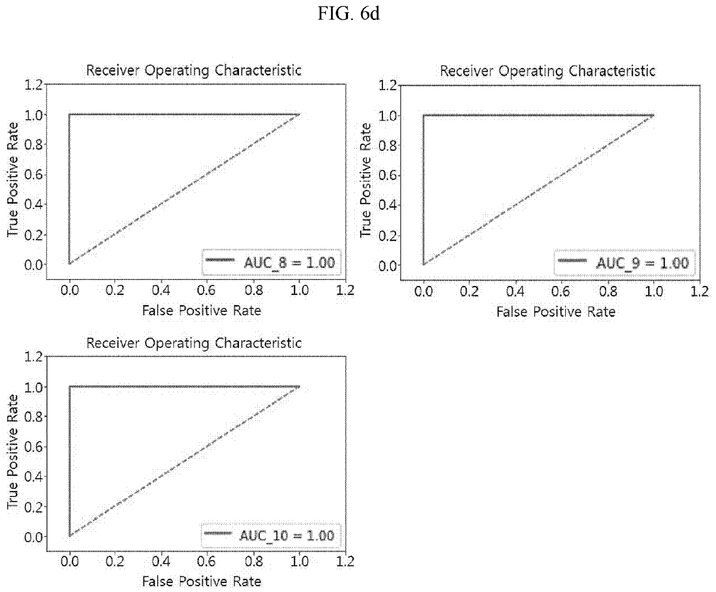

As a result of the analysis of Example 5-2, through both regression analysis and random forest analysis, it was confirmed that the performance of predicting the tumor reactivity in the case in which the number of interactions is 3, that is, a model consisting of the combination of three genes was the highest, and the inventors plotted ROC curves for each of 10 features showing a positive coefficient value, which consists of three types of genes. FIGS. 6A and 6B show tables of 10 gene combinations and ROC curves for all of 10 features, and FIGS. 6C and 6D show ROC curves plotted only using the cases in which AUC values are 0.7 or more when there is a positive coefficient value (Combinations 2, 5, 6, 8, 9 and 10).

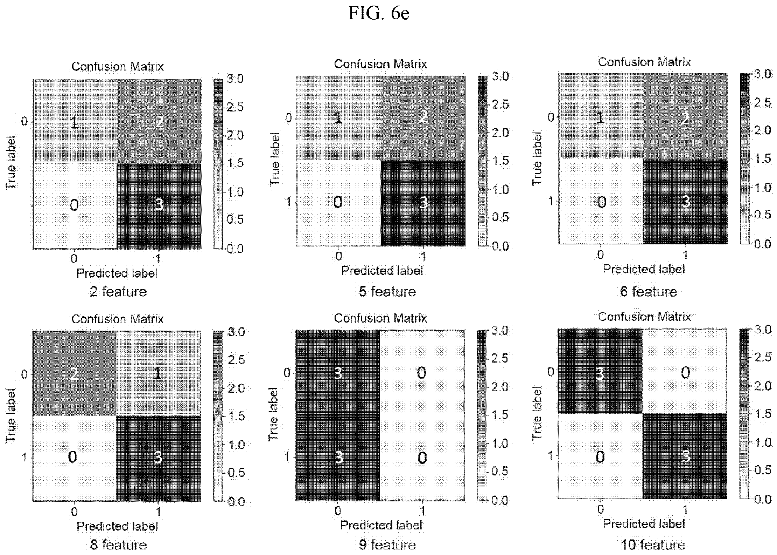

Further, the inventors plotted a confusion matrix for each of 6 features having an AUC value of 0.7 or more to compare the performances of models consisting of each combination. As a result, as shown in FIG. 6e, it was confirmed that, features 8 and 10 matched all three reactivity(marked as 1), and features 8 and 10 had higher accuracy than other features in non-reactivity(marked as 0).

Therefore, from the results, Combination 8 (ATP6VOA1*TSPAN2*MBOAT2) and Combination 10 (PTPN13*TCN2*TSPAN2) were finally selected as significant combinations of marker genes, which are to predict the tumor reactivity of lymphocytes.

It should be understood by those of ordinary skill in the art that the above descriptions of the present invention are exemplary, and the embodiments disclosed herein can be easily modified into other specific forms without changing the technical spirit or essential features of the present invention. Therefore, it should be interpreted that the embodiments described above are exemplary in all aspects, and are not limitative.

### [Industrial Applicability]

By using the genetic marker according to the present invention, it is possible to separate lymphocytes from body tissues, blood, or body fluids more conveniently by non-invasive method rather than the conventional invasive method, and predict their tumor reactivity to select tumor-specific lymphocytes. And based on this, an effective immunotherapeutic agent may be produced, so a lymphocyte selection marker having tumor-specific reactivity is expected to be widely used in the field of immunotherapy.

## Claims

1. A composition for predicting a tumor reactivity of lymphocyte, comprising
an agent capable of measuring a mRNA level or an agent capable of measuring a protein level for ITGA6 gene (Genbank accession number: NM_000210.4, NM 001079818.3, NM 001316306.2, NM 001365529.2 or NM_00 1365530.2).

2. The composition of claim 1, wherein the composition further comprises an agent capable of measuring a mRNA level or an agent capable of measuring a protein level for at least one gene selected from the group consisting of
ATP6V0A1 (Genbank accession number: NM 001130020.3 NM 001130021.3 NM 001378522.1NM_001378523.1 or NM_001378530.1),
ARRDC3(Genbank accession number: NM 001329670.2, NM 001329671.2, NM_001329672.2 and NM_020801.4),
CD23(Genbank accession number: NM 001207019.2, NM 001220500.2 or NM 002002.4),
CD200( Genbank accession number: NM_001004196.3, NM 001318826.1, NM 001318828.1, NM 001318830.1 or NM_001365851.2),
CD300C(Genbank accession number: NM 006678.5),
CYSLTR1(Genbank accession number: NM_001282186.1 NM_001282187.2 NM_001282188.2 or NM 006639.4),
ITGB1(Genbank accession number: NM_002211.4, NM_033668.2 or NM_133376.2),
MBOAT2(Genbank accession number: NM 001321265.2, NM 001321266.2, NM_001321267.2 or NM_138799.4),
Met(Genbank accession number: NM 000245.4, NM 001127500.3, NM 001324401.2 or NM_001324402.2),
MYO9A(Genbank accession number: NM 006901.4),
PTPN13(Genbank accession number: NM_006264.3, NM_080683.3, NM_080684.3 or NM_080685.2),
S100P( Genbank accession number: NM 005980.3),
SECTM 1 (Genbank accession number: NM_003004.3),
TCN2( Genbank accession number: NM 000355.4 or NM 001184726.1),
TSPAN2(Genbank accession number:NM_001308315.1, NM_001308316.1 or NM_005725.6) and
VSIG1(Genbank accession number: NM 001170553.1 or NM_182607.5).

3. The composition of claim 1,
wherein the lymphocyte is isolated from tumor tissue, blood or body fluid.

4. The composition of claim 1 or 2,
wherein the agent capable of measuring the mRNA level is a sense primer, an antisense primer or probe, which are capable of complementarily binding to the mRNA of the gene.

5. The composition of claim 1 or 2,
wherein the agent capable of measuring the protein level is an antibody that specifically binds to the protein encoded by the gene.

6. A composition for predicting a tumor reactivity of lymphocyte, comprising an agent capable of measuring a mRNA level or an agent capable of measuring a protein level for three or more genes selected from the group consisting of
ATP6V0A1(Genbank accession number: NM 001130020.3, NM_001 130021.3, NM_001378522.1, NM_001378523.1 or NM 001378530.1),
MBOAT2(Genbank accession number: NM_001321265.2, NM_001321266.2, NM_001321267.2 or NM 138799.4),
PTPN13(Genbank accession number: NM_006264.3, NM_080683.3, NM_080684.3 or NM_080685.2),
TCN2(Genbank accession number: NM 000355.4 or NM_001184726.1) and
TSPAN2( Genbank accession number: NM 001308315.1, NM 001308316.1 or NM 005725.6).

7. The composition of claim 6,
wherein the composition comprises the agent capable of measuring a mRNA level or the agent capable of measuring a protein level for ATP6V0A1, MBOAT2 and TSPAN2 genes.

8. The composition of claim 6,
wherein the composition comprises the agent capable of measuring a mRNA level or the agent capable of measuring a protein level for PTPN13, TCN2 and TSPAN2 genes.

9. The composition of claim 6,
wherein the composition further comprises an agent capable of measuring a mRNA level or an agent capable of measuring a protein level for at least one gene selected from the group consisting of
ARRDC3(Genbank accession number: NM_001329670.2, NM_001329671.2, NM_00 1329672.2 or NM 020801.4),
CD23(Genbank accession number: NM_001207019.2, NM_001220500.2 or NM_002002.4 ),
CD200(Genbank accession number: NM 001004196.3, NM_001318826.1, NM_001318828.1, NM_001318830.1 or NM 001365851.2),
CD300C(Genbank accession number: NM_006678.5),
CYSLTR1(Genbank accession number: NM 001282186.1, NM 001282187.2, NM 001282188.2 or NM_006639.4),
ITGA6(Genbank accession number: NM 000210.4, NM 001079818.3, NM 001316306.2, NM_001365529 .2 or NM_001365530.2),
ITGB 1 (Genbank accession number: NM 002211.4, NM 033668.2 or NM_133376.2), Met(Genbank accession number: NM_000245.4, NM 001127500.3, NM 001324401.2 or NM 001324402.2),
MYO9A(Genbank accession number: NM_006901.4),
S100P(Genbank accession number:NM_005980.3),
SECTM 1 (Genbank accession number: NM 003004.3) and
VSIG1 (Genbank accession number: NM_001170553.1 or NM 182607.5).

10. The composition of claim 6,
wherein the lymphocyte is isolated from tumor tissue, blood or body fluid.

11. The composition according to any one of claims 6 to 9,
wherein the agent capable of measuring the mRNA level is a sense primer, an antisense primer or probe, which are capable of complementarily binding to the mRNA of the gene.

12. The composition according to any one of claims 6 to 9,
wherein the agent capable of measuring the protein level is an antibody that specifically binds to the protein encoded by the gene.

13. A method for predicting a tumor reactivity of lymphocyte, the method comprises
measuring a mRNA level or a protein level for ITGA6 gene (Genbank accession number: NM 000210.4, NM_001079818.3, NM_001316306.2, NM_001365529.2 or NM 001365530.2)

14. A method for predicting a tumor reactivity of lymphocyte, the method comprises
measuring a mRNA level or a protein level for three or more genes selected from the group consisting of
ATP6V0A1(Genbank accession number: NM 001130020.3, NM_001 130021.3, NM_001378522.1, NM_001378523.1 or NM 001378530.1),
MBOAT2(Genbank accession number: NM_001321265.2, NM_001321266.2, NM_001321267.2 or NM 138799.4),
PTPN13(Genbank accession number: NM_006264.3, NM_080683.3, NM_080684.3 or NM_080685.2),
TCN2(Genbank accession number: NM 000355.4 or NM_001184726.1) and
TSPAN2( Genbank accession number: NM 001308315.1, NM 001308316.1 or NM 005725.6).
